# EUROPEAN PATENT APPLICATION

(11) **EP 3 791 854 A1**
(43) Date of publication of application: **17.03.2021**
(21) Application number: 20195182.9
(22) Date of filing: 09.09.2020
(51) Int. Cl.: A61K 8/35, A61Q 5/00, A61Q 17/00

(54) **USE OF COMPOSITIONS COMPRISING DIHYDROXYACETONE FOR THE PROTECTION OF SKIN OR HAIR AGAINST INFRARED IRRADIATION**

(30) Priority: 13.09.2019 EP 19197246
(71) Applicant: Merck Patent GmbH, 64293 Darmstadt (DE)
(72) Inventor: Bicard Benhamou, Valerie, 64297 DARMSTADT (DE); Heider, Lilia, 64579 GERNSHEIM (DE); Lefort, Marina, 64291 Darmstadt (DE); Zur Lage, Jutta, 64285 DARMSTADT (DE)

(57) **Abstract**

The present invention relates to the non-therapeutical, cosmetic use of dihydroxyacetone in a cosmetic composition to be applied on skin, scalp, or hair for the protection of skin, scalp, or hair against infrared irradiation as well as the use of a cosmetic composition comprising dihydroxyacetone as an agent for controlling tropoelastin expression in living cells of the epidermis and papillary dermis or as an agent for controlling the degradation of extracellular matrix components in living cells of the epidermis and papillary dermis. It further relates to dihydroxyacetone as an active ingredient to protect skin, scalp, or hair against damages after infrared irradiation by reacting with the skin, scalp, or hair or by penetrating the skin, scalp, or hair.

## Description

### Field of the Invention

The present invention relates to the non-therapeutical, cosmetic use of dihydroxyacetone in a cosmetic composition to be applied on skin, scalp, or hair for the protection of skin, scalp, or hair against infrared irradiation as well as the use of a cosmetic composition comprising dihydroxyacetone as an agent for controlling tropoelastin expression in living cells of the epidermis and papillary dermis or as an agent for controlling the degradation of extracellular matrix components in living cells of the epidermis and papillary dermis. The invention relates further to the use of dihydroxyacetone for the preparation of topical dermatological or pharmaceutical compositions or medical devices to be applied on skin, scalp, or hair for the protection of skin, scalp, or hair against damages of infrared irradiation. It further relates to dihydroxyacetone as an active ingredient to protect skin, scalp, or hair against damages after infrared irradiation by reacting with the skin, scalp, or hair or by penetrating the skin, scalp, or hair.

### Background of the Invention

The epidermis, which is the outermost layer of the skin is primarily composed of keratinocytes. These cells differentiate upward form the basement membrane (*stratum basale*), where they are in contact with the dermis, to form stratified layers which end as sheets of connected cornified cells at the outermost position.

The dermis or corium is a layer of skin between the epidermis and subcutaneous tissues, that primarily consists of dense irregular connective tissue. It is divided into two layers, the superficial area adjacent to the dermis called papillary dermis and a deep thicker area known as the reticular dermis. The papillary dermis is made up of fine collagen fibers that are arranged in a loose fashion.

Skin, scalp, and hair are frequently exposed to external conditions. One kind of external condition is the exposition with electromagnetic irradiation. It remains in minds that photoprotection is mainly a matter of UV protection. However, all solar radiations lead to formation of radical oxygen species (ROS) and excess of free radicals in skin contribute to premature skin aging and wrinkling. Visible light and especially high energy visible light (HEVL) notably trigger pro-inflammatory cytokines, different matrix metalloproteinases expression, or oxidation of proteins and these markers all play a role in accelerating skin aging e.g. sagging skin, inflammation, wrinkles and pigmentation disorders (Liebel F., Kaur, S., Ruvolo E., Kollias N., Southall M.D., Journal of Investigative Dermatology, 2012, 132, 1901-1907).

In comparison, infrared (IR) radiation has the lowest energy level but its contribution to the solar spectrum reaching the human skin is in the range of 50% and according to Schroeder et al., Free Radical Biol, 2007, 43, 128-135 about 53%. Therefore, the biological impact on the skin may be strong as explained in Shaath, N, "Infrared radiation skin protection", HAPPI, 2012, pp. 2-5.

IR light and particularly IR-A with wavelength of 700 nm to 1400 nm according to the International Commission on Illumination (CIE), induce significant free radicals in the dermis and diminish the skin's antioxidant capacity as described in Holzer A., Athar, M and Elmets, C, "The other end of the rainbow: infrared and skin", J. Invest. Dermatol., 2010, 130, pp. 1496-1498. At high IR dosis, harmful effects are reported in Akhalaya, MY, Maksimov, GV, Rubin, AB, Lademann, J, Darvin ME, "Molecular action mechanisms of solar infrared radiation and heat on human skin", Ageing Research Reviews, 2014, 16, pp. 1-11. It is further stated in this last citation that IR radiation has been shown to alter the collagen content of the dermal extracellular matrix (ECM) not only by leading to an increased expression of the collagen degrading enzymes MMPs (matrix metalloproteinases), but also by decreasing the *de novo* collagen synthesis. It is further described that the mechanisms of the amplification of MMPs synthesis in the skin during IR irradiation are different in comparison with the action of UV radiation. The following differences are discussed on page 3, column 2:
a) Formation of ROS after IR exposure occurs in the inner membrane of the fibroblast mitochondria while UV irradiation targets the mitochondrial DNA and its alteration leading to an increased ROS formation;
b) Antioxidant treatment of fibroblasts in mitochondria inhibits the formation of ROS after IR, but not after UV irradiation;
c) The blockage of the electron transfer in the mitochondrial ETC prevents a response to the IR effect, but not to that of UV irradiation;
d) Dermal fibroblasts of high respiratory activity are more sensitive to IR radiation while this effect is not observed during UV irradiation.

The matrix metalloproteinases are a family of peptidase enzymes responsible for the degradation of extracellular matrix components, including collagen, gelatin, fibronectin, laminin and proteoglycan. Matrix metalloproteinase-1 (MMP-1) is an enzyme also known as intersitial collagenase and fibroblast collagenase. Schroeder et al, Free Radical Biology & Medicine, 2007, 43, 128-135 describes that IR-A radiation elicits a retrograde signaling response, which is initiated in the mitochondria through generation of reactive oxygen species that originate from the mitochondrial electron transport chain.

Elastin is the major component of the elastic fibers of the skin. It is a protein of the connective tissue responsible for the elastic properties of the skin. It is initially synthesized as its precursor, tropoelastin. Deposition of tropoelastin into the extracellular space occurs only at specific regions on the cell surface, and tropoelastin is rapidly incorporated into the forming elastic fiber. Chen et al, "Modulation of tropoelastin and fibrillin-1 by infrared radiation in human skin in vivo", Photoderm. Photoimmun, Photomed. 2009, 25 (6), pp. 310-316 describes recently the impact of infrared on tropoelastin expression at mRNA and protein levels.

Further information on the effects of infrared radiation on skin can be found in Soyun Cho et al, "Effects of Infrared Radiation and Heat on Human Skin Aging in vivo", Journal of Investigative Dermatology Symposium Proceedings, 2009, 14, pp. 15-19. It has been reported therein that IR-A can penetrate epidermal and dermal layers and reach subcutaneous tissues without increasing the skin temperature significantly.

There are already powerful solutions to address light protection beyond UV, notably in the HEV and IR-A ranges, considering the first defense line strategy, meaning avoiding any radiations to enter the skin e.g. by using appropriate titanium dioxide UV filters.

In the second defense strategy, ingredients help reduce the overall radical burden on the skin, modulate biochemical markers in a way that damages on the skin can be minimized.

Dihydroxyacetone (DHA) is a recognized ingredient in cosmetic for its strong self-tanning properties. It is a colorless solid with characteristic odor. In freshly prepared aqueous solutions, it is present as dimer which segments into the monomers by heating within approximately 30 minutes (T. Kurz, Cosmetics & Toiletries magazine, 1994, 109, 11, 55).

Self-tanning cosmetics work to color the skin by the reaction of the skin and dihydroxyacetone (1,3-dihydroxy-2-propanone) contained in the cosmetic composition thereof. Self-tanning agents such as DHA can react with the proteins and amino acids of the horny layer of the skin in the sense of a Maillard reaction or via a Michael addition, where polymers result which give the skin a brownish hue form via a reaction route which has not yet been clarified completely. This reaction is complete after about 4 to 6 hours. The tan achieved in this way cannot be washed off and is only removed with the normal skin desquamation.

EP1092415 describes cosmetic and dermatological preparations for tanning the skin, in particular those which also offer protection against UV radiation.

EP1277461 describes sunscreen compositions comprising DHA as self-tanning agent.

K.A. Follett et al, Dermatologica, 1987, 58-63; J. A. Johnson et al, Dermatologica, 1987, 53-57 and J.A. Johnson, British Journal of Dermatology, 1992, 126, 2, 94 describe that the self-tanning agent DHA is able to protect against long wavelength UV-A radiation (320 to 400 nm) and/or blue light. However, it is generally known that the protection against UV-A rays achieved through tanning of the skin with the aid of DHA is not as pronounced as the protection effected by sun tanning.

US7311895 describes cosmetic formulations containing DHA and an osmolyte such as ectoine wherein such cosmetic formulations are characterized in that the UV-A protective effect of dihydroxyacetone is increased.

KR19970015683 describes pigments which are surface-treated with dihydroxyacetone and amino acids reacting with light of 400 to 500 nm.

However, there is still a strong need to have diversified solutions for the protection of skin, scalp, and hair due to infrared (IR) irradiation.

Consequently, it is an object of the present application to provide such a solution.

### Summary of the Invention

The present inventors have now found that the above object may be attained by applying dihydroxyacetone on skin, scalp, or hair through a cosmetic, dermatological or pharmaceutical composition or a medical device able to be applied topically on skin, scalp, or hair.

The invention relates to a non-therapeutic, cosmetic use of dihydroxyacetone in a cosmetic composition to be applied on skin, scalp, or hair for the protection of skin, scalp, or hair against infrared irradiation.

The invention relates further to a use of dihydroxyacetone for the preparation of topical dermatological or pharmaceutical compositions or medical devices to be applied on skin, scalp, or hair for the protection of skin, scalp, or hair against damages of infrared irradiation.

The invention relates further to dihydroxyacetone as an active ingredient to protect skin, scalp, or hair against damages after infrared irradiation by reacting with the skin, scalp, or hair.

The invention relates further to dihydroxyacetone as an active ingredient to protect skin, scalp, or hair against damages after infrared irradiation by penetrating the skin, scalp, or hair.

### Detailed Description of the Invention

As disclosed before, by reaction of dihydroxyacetone with skin, scalp, or hair or by penetrating the skin, scalp, or hair to protect said anatomical areas, there is no restriction with regard to skin-type or the origin of said skin or hair. Preferably, the invention relates to human skin, scalp, or hair. Particular preferably, the invention relates to human skin.

The protective effect of DHA against infrared radiations, preferably against IR-A radiation between 700 nm and 1400 nm, particularly preferably against IR-A radiation between 700 nm and 1150 nm, is surprisingly found while investigating the properties of dihydroxyacetone on living human skin explants.

For the purposes of the invention, dihydroxyacetone is defined such that it is also taken to mean conformers, or dimers, or solvates.

Solvates of DHA are taken to mean adductions of inert solvent molecules onto the compound which form, owing to their mutual attractive force.

The conformation of an organic molecule describes the spatial arrangement of its rotatable bonds at the carbon atoms. It fully describes the three-dimensional spatial coordinates of all atoms of the molecule. Molecules having the same arrangement of atoms, but which differ in the specific arrangement of the atoms and lie at an energy minimum, are called conformers. The term rotamer is also common as a synonym therefor.

For the preparation of cosmetic, dermatological, or pharmaceutical compositions or medical devices to be used according to the invention, DHA is preferably used in its powder form.

The powder form of crystalline or amorphous DHA corresponds to at least one dimeric form. Usually, the solid DHA is a mixture of dimeric forms. The following dimers are known which can be used according to the invention:

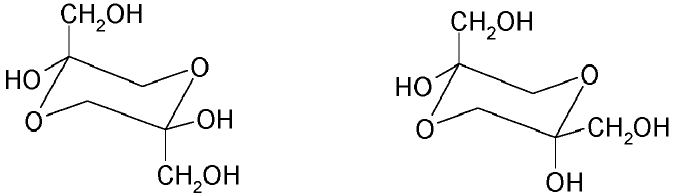

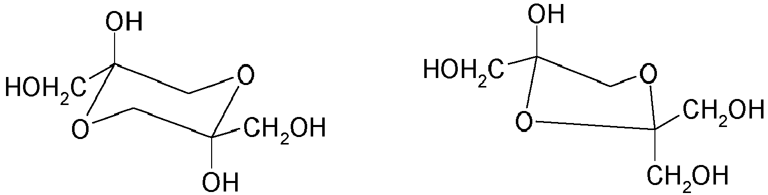

The structure of DHA in its monomeric form is the following:

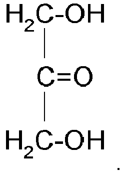

Either form of DHA once applied to skin via a cosmetic, dermatological, or pharmaceutical composition or medical device may react with the proteins and amino acids of skin, scalp, or hair for a protection against infrared irradiation, preferably for protection against IR-A irradiation between 700 nm and 1400 nm, particular preferably for protection against IR-A irradiation between 700 nm and 1150 nm.

For said non-therapeutic, cosmetic use, DHA reacting with skin, scalp, or hair can protect these anatomical areas against said irradiation to prevent skin, scalp, or hair to become damaged by said irradiation.

For said non-therapeutic, cosmetic use, DHA as active ingredient can protect skin, scalp, or hair against damages after infrared irradiation by penetrating the skin, scalp, or hair.

The invention is therefore further directed to the use of DHA in a cosmetic, dermatological, or pharmaceutical preparation or in a medical device, as described before, wherein dihydroxyacetone protects skin, scalp, or hair against IR-A irradiations between 700 nm and 1400 nm, preferably human skin, scalp, or hair.

The invention is therefore further directed to the use of DHA in a cosmetic, dermatological, or pharmaceutical preparation or in a medical device, as described before, wherein dihydroxyacetone protects skin, scalp, or hair against IR-A irradiations between 700 nm and 1150 nm, preferably human skin, scalp, or hair.

The invention is further directed to DHA as an active ingredient to protect skin, scalp, or hair against damages after IR-A irradiation between 700 nm and 1400 nm by reacting with skin, preferably with human skin.

The invention is further directed to DHA as an active ingredient to protect skin, scalp, or hair against damages after IR-A irradiation between 700 nm and 1150 nm by reacting with skin, preferably with human skin.

The invention is further directed to DHA as an active ingredient to protect skin, scalp, or hair against damages after IR-A irradiation between 700 nm and 1400 nm by penetrating skin, preferably human skin.

The invention is further directed to DHA as an active ingredient to protect skin, scalp, or hair against damages after IR-A irradiation between 700 nm and 1150 nm by penetrating the skin, preferably human skin.

The invention is furthermore preferably carried out on said part of human skin, which builds the epidermis and the papillary dermis.

For the purposes of the present invention, the term "composition" is also used synonymously alongside the term "preparation".

Generally, there are no restrictions with regard to the cosmetic, dermatological, or pharmaceutical preparation or medical device comprising DHA in accordance with the present invention. Preferred compositions are disclosed in the following.

The preparations used according to the invention here are preparations which can be applied topically. "Can be applied topically" means that the preparation is applied externally and locally, i.e. that the preparation must be suitable, for example, for being able to be applied to the skin, scalp, or hair so that DHA is able to react with said skin, scalp, or hair forming the active ingredient as described before.

Usually, the preparations comprise a cosmetically, pharmaceutically or dermatologically suitable vehicle and, depending on the desired property profile, optionally further suitable ingredients. The topical preparations are preferably employed as cosmetic or dermatological composition, particularly preferably as cosmetic composition. Suitable vehicles and assistants or fillers are described in detail in the following part.

Combination of DHA as described before or preferably described before, with other active ingredients to further support such protective ability in a cosmetic, dermatological, or pharmaceutical composition or medical device or to further support the general state of skin, scalp, or hair is advantageous.

Particularly suitable further ingredients in the composition comprising DHA are fillers containing boron nitride, synthetic sapphire or bismuth oxychloride. It is believed that such fillers may support the activity of DHA in an advantageous manner.

The filler should preferably be used in 0.5% by weight to 5% by weight, preferably by 3% by weight when combined with DHA.

Very suitable fillers contain synthetic sapphire, e.g. marketed as RonaFlair® White Sapphire. Very suitable fillers contain bismuth oxychloride, e.g. marketed as RonaFlair® B-50, RonaFlair® Fines or RonaFlair® LF-2000. Very suitable fillers contain boron nitride, e.g. marketed as RonaFlair® Boroneige, SF-3 and RonaFlair® Boroneige, SF-15.

The preparations described, which in accordance with the invention comprise DHA as described before or preferably described before, may furthermore also comprise coloured pigments, where the layer structure of the pigments is not limited.

The coloured pigment should preferably be on use of 0.5% by weight to 5% by weight, preferably of 1% by weight to 3% by weight. The selection of a corresponding pigment is familiar to the person skilled in the art. Very suitable coloured pigments are pearlescent pigments. Very suitable coloured pigments are pearlescent pigments. Preferred pearlescent pigments are marketed as Colorona® Red Gold, Ronastar® Silver, Ronastar® Noble Sparks, Ronastar® Copper Sparks, Ronastar® Golden Sparks, Ronastar® Golden Lights, Timiron® Liquid Silver, Timiron® Starluster MP-115, Xirona® Volcanic Fire. Such pigments are particularly suitable further ingredients in the composition comprising DHA.

Particularly suitable active ingredients for combination are humectants. Suitable humectants to be used together with the mixture according to the invention in a cosmetic, dermatological, or pharmaceutical composition or medical device are ectoine (RonaCare®Ectoin), hydroxyectoine, trehalose, glycerol, glycosyl glycerol, β-mannosyl glycerate (firoin), β-mannosylglyceramide (firoin A), di-myo-inositol phosphate (DIP), cyclic 2,3-diphosphoglycerate (cDPG), 1,1-diglycerol phosphate (DGP), dimannosyl diinositol phosphate (DMIP), betaine, glycine betaine, proline betaine, glutamate betaine, alanine, proline, glutamine, N-acetyl lysine, glutamine 1-amide, taurine, choline, choline O-sulfate, carnitine, arsenobetaine, crotonobetaine, dimethyl sulfonioacetate, dimethyl sulfopropionate, homobetaine, trimethylamine N-oxide, panthenol, sorbitol, meglumine, hyaluronic acid or hyaluronic acid derivatives, urea (RonaCare®Urea), and niacinamide (RonaCare®Nicotinamide).

The preparations may include or comprise, essentially consist of or consist of the necessary or optional constituents mentioned above and/or below. All compounds or components which can be used in the preparations are either known and commercially available or can be synthesised by known processes.
The preparation is preferably a cosmetic or dermatological preparation; the preparation is particularly preferably a cosmetic preparation.

Dihydroxyacetone as described before is employed in the topical preparations in amounts of 0.01 to 20% by weight, preferably in amounts of 0.05 to 15% by weight, particularly preferably in amounts of 0.1% by weight to 8% by weight and very particularly preferably in amounts of 0.5 to 3% by weight, based on the total amount of the preparation.

Preparations for use according to the invention comprising DHA, tend towards malodours on application to the human skin, which are thought to be caused by degradation products of dihydroxyacetone itself or by products of side reactions and which are regarded as unpleasant by some users. It has been found that these malodours are prevented on use of formaldehyde scavengers and/or flavonoids. The preparation to be used according to the invention may therefore preferably also comprise formaldehyde scavengers and optionally flavonoids for improving the odour.

The formaldehyde scavenger is preferably selected from the group alkali metal, alkaline-earth metal or ammonium disulfite. Particular preference is given to a preparation which comprises, in combination DHA Plus, a mixture of DHA, sodium disulfite and magnesium stearate. DHA Plus is marketed by Merck KGaA, Darmstadt.

The preparation to be used according to the invention comprising DHA, may particularly preferably comprise flavonoids.

The flavonoid here additionally acts as stabiliser for the self-tanner or the self-tanning substances and/or reduces or prevents or improves storage-dependent malodours, which may also arise due to additives or assistants present.

This is preferably a flavonoid in which one or more phenolic hydroxyl groups have been blocked by etherification or esterification. For example, hydroxyethyl-substituted flavonoids, such as, preferably, troxerutin, troxequercetin, troxeisoquercetin or troxeluteolin, and flavonoid sulfates or flavonoid phosphates, such as, preferably, rutin sulfates, have proven to be particularly highly suitable flavonoids here. In the sense of this use, particular preference is given to rutin sulfate and troxerutin. Very particular preference is given to the use of troxerutin.

The preferred flavonoids have a non-positively charged flavan skeleton. It is thought that metal ions, such as, for example, Fe²⁺/Cu²⁺, are complexed by these flavonoids and auto-oxidation processes in the case of fragrances or compounds whose degradation results in malodours are thus prevented or reduced.

Particular preference is given to the mixture of DHA with troxerutine, called DHA Rapid, marketed from Merck, Darmstadt.

Furthermore, the topical preparations to be used according to the invention may comprise at least one further humectant as further ingredient as described before. Such further humectant(s) is/are preferably present in the topical preparation in an amount of 0.01 to 20% by weight, particularly preferably in an amount of 0.1 to 15% by weight and very particularly preferably in an amount of 0.2 to 8% by weight, based on the total amount of the preparation.

Besides DHA, formaldehyde scavengers, flavonoids or humectants, as described above or described as preferred, the preparations may additionally also comprise at least one UV filter.
Organic UV filters, so-called hydrophilic or lipophilic sun-protection filters, are effective in the UVA region and/or UVB region and/or IR and/or VIS region (absorbers). These substances can be selected, in particular, from dibenzoylmethane derivatives, cinnamic acid derivatives, salicylic acid derivatives, camphor derivatives, triazine derivatives, β,β-diphenytacrytate derivatives, p-aminobenzoic acid derivatives and polymeric filters and silicone filters, which are described in the application WO-93/04665. Further examples of organic filters are indicated in the patent application EP-A 0 487 404. The said UV filters are usually named below in accordance with INCI nomenclature.

Particularly suitable for a combination with DHA are Ethylhexyl salicylate, Phenylbenzimidazolesulfonic acid and its salts, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Butyl Methoxydibenzoylmethane, Ethylhexyl Triazone, Diethylhexyl Butamido Triazone, Drometrizole trisiloxane, Phenylene bis-diphenyltriazine, Polysilicone-15, 1,1-Dicarboxy(2,2'-dimethylpropyl)-4,4-diphenylbutadiene, 2,4-bis[5-1 (dimethylpropyl)benzoxazol-2-yl(4-phenyl) imino]-6-(2-ethylhexyl)imino-1,3,5-triazine and mixtures thereof. These organic UV filters are generally incorporated into preparations in an amount of 0.01 % by weight to 20% by weight, preferably 1% by weight to 10% by weight.

Besides the ingredients beside DHA, as described above, the preparations may comprise further inorganic UV filters, so-called particulate UV filters.

The following examples are also encompassed by the present disclosure and may fully or partly be incorporated into embodiments.
Preference is given here both to those from the group of the titanium dioxides, such as, for example, coated titanium dioxide (for example Eusolex® T-2000, Eusolex®T-AQUA, Eusolex®T-AVO, Eusolex®T- PRO, preferably Eusolex® T-EASY), zinc oxides (for example Sachtotec®), iron oxides or also cerium oxides and/or zirconium oxides.
It may furthermore be preferred for the preparations to comprise inorganic UV filters which have been aftertreated by conventional methods, as described, for example, in Cosmetics & Toiletries, February 1990, Vol. 105, pp. 53 64. One or more of the following aftertreatment components can be selected here: amino acids, beeswax, fatty acids, fatty acid alcohols, anionic surfactants, lecithin, phospholipids, sodium, potassium, zinc, iron or aluminium salts of fatty acids, polyethylenes, silicones, proteins (particularly collagen or elastin), alkanolamines, silicon dioxide, aluminium oxide, further metal oxides, phosphates, such as sodium hexametaphosphate, or glycerine.

These inorganic UV filters are generally incorporated into the preparations in an amount of 0.1% by weight to 25% by weight, preferably 2% by weight to 10% by weight.

By combination of one or more of the said compounds having a UV filter action, the protective action against harmful effects of both UV radiation and IR radiation inducing photo-ageing can be optimised.

All said UV filters can also be employed in encapsulated form. In particular, it is advantageous to employ organic UV filters in encapsulated form.

The capsules in said topical preparations are preferably present in amounts which ensure that the encapsulated UV filters are present in the preparation in the per cent by weight ratios indicated above.

Further preparations comprising DHA as described before or preferably described before may likewise comprise at least one further cosmetic active compound, for example selected from antioxidants, anti-ageing, antiwrinkle, anti-dandruff, anti-acne, anti-cellulite active compounds, deodorants or vitamins.

The protective action of said preparations against oxidative stress or against the effect of free radicals can be improved if the preparations comprise one or more antioxidants, the person skilled in the art being presented with absolutely no difficulties in selecting antioxidants which act suitably quickly or with a time delay.

There are many proven substances known from the specialist literature which can be used as antioxidants, for example amino acids (for example glycine, tyrosine, tryptophan) and derivatives thereof, imidazoles, (for example urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, lipoic acid and derivatives thereof (for example dihydrolipoic acid), aurothioglucose, propylthiouracil and other thiols (for example thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (for example buthionine sulfoximines, homocysta sulfoximine, buthionine sulfones, penta-, hexa- and heptathionine sulfoximine) in very low tolerated doses (for example pmol to µmol/kg), and also (metal) chelating agents, (for example α-hydroxyfatty acids, palmitic acid, phytic acid, lactoferrin), α-hydroxy acids (for example citric acid, lactic acid, malic acid), humic acid, bile acid, bile extracts, bilirubin, biliverdin, EDTA, EGTA, pentasodium ethylenediamine tetramethylene phosphonate and derivatives thereof, unsaturated fatty acids and derivatives thereof, vitamin C and derivatives (for example ascorbyl palmitate, magnesium ascorbyl phosphate, ascorbyl acetate), tocopherols and derivatives (for example vitamin E acetate), vitamin A and derivatives (for example vitamin A palmitate) and coniferyl benzoate of benzoin resin, rutinic acid and derivatives thereof, α-glycosylrutin, ferulic acid, furfurylideneglucitol, carnosine, butylhydroxytoluene, butylhydroxyanisole, nordihydroguaiaretic acid, trihydroxybutyrophenone, quercetin, uric acid and derivatives thereof, mannose and derivatives thereof, zinc and derivatives thereof (for example ZnO, ZnSO₄), selenium and derivatives thereof (for example selenomethionine), stilbenes and derivatives thereof (for example stilbene oxide, trans-stilbene oxide).

Suitable antioxidants are also compounds of the formulae A or B 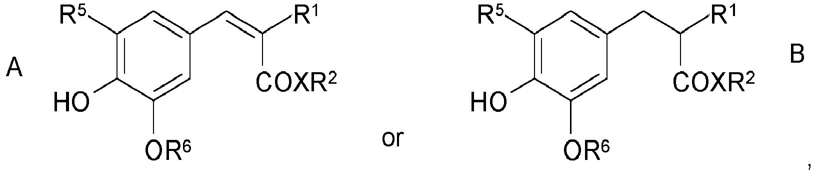 in which
- R¹: can be selected from the group -C(O)CH₃, -CO2R₃, -C(O)NH₂ and -C(O)N(R⁴)₂,
- X: denotes O or NH,'
- R²: denotes linear or branched alkyl having 1 to 30 C atoms,
- R³: denotes linear or branched alkyl having 1 to 20 C atoms,
- R⁴: in each case, independently of one another, denotes H or linear or branched alkyl having 1 to 8 C atoms,
- R⁵: denotes H, linear or branched alkyl having 1 to 8 C atoms or linear or branched alkoxy having 1 to 8 C atoms and
- R⁶: denotes linear or branched alkyl having 1 to 8 C atoms,
preferably derivatives of 2-(4-hydroxy-3,5-dimethoxybenzylidene)malonic acid and/or 2-(4-hydroxy-3,5-dimethoxybenzyl)malonic acid, particularly preferably bis(2-ethylhexyl) 2-(4-hydroxy-3,5-dimethoxybenzylidene)malonate (for example Oxynex®ST Liquid) and/or bis(2-ethylhexyl) 2-(4-hydroxy-3,5-dimethoxybenzyl)malonate (for example RonaCare®AP).

Mixtures of antioxidants are likewise suitable for use in the cosmetic preparations according to the invention. Known and commercial mixtures are, for example, mixtures comprising, as active ingredients, lecithin, L-(+)-ascorbyl palmitate and citric acid, natural tocopherols, L-(+)-ascorbyl palmitate, L-(+)-ascorbic acid and citric acid (for example Oxynex® K LIQUID), tocopherol extracts from natural sources, L-(+)-ascorbyl palmitate, L-(+)-ascorbic acid and citric acid (for example Oxynex® L LIQUID), DL- α-tocopherol, L-(+)-ascorbyl palmitate, citric acid and lecithin (for example Oxynex® LM) or butylhydroxytoluene (BHT), L-(+)-ascorbyl palmitate and citric acid (for example Oxynex® 2004). Antioxidants of this type are usually employed in such preparations with the mixture according to the invention in per cent by weight ratios in the range from 1000:1 to 1:1000, preferably in per cent by weight ratios of 100:1 to 1:100.

Suitable anti-ageing active compounds, in particular for skin-care preparations, are products from Merck, such as, for example, 5,7-dihydroxy-2-methylchromone, marketed under the trade name RonaCare®Luremin®, or the commercial products RonaCare®ASCIII®, RonaCare®RenouMer, RonaCare®VTA, RonaCare®Poppy SE, RonaCare®lsoquercetin, RonaCare®Tilirosid, RonaCare®Cyclopeptide 5, RonaCare®Cyclopeptide 5 alcoholfree.

The preparations to be employed may comprise the antimicrobially active compounds described in WO 2013/091775 A2, WO 2013/159865 A1 or WO 2013/167220 A1, in particular 4-hydroxy-cyclohexanecarboxylic acid butyl ester (available as RonaCare®SereneShield from Merck KGaA, Darmstadt, Germany).

The preparations to be employed may comprise vitamins as further ingredients. Preference is given to vitamins and vitamin derivatives selected from vitamin A, vitamin A propionate, vitamin A palmitate, vitamin A acetate, retinol, vitamin B, thiamine chloride hydrochloride (vitamin B₁), riboflavin (vitamin B₂), nicotinamide, vitamin C (ascorbic acid), vitamin D, ergocalciferol (vitamin D₂), vitamin E, DL-α-tocopherol, tocopherol E acetate, tocopherol hydrogensuccinate, vitamin K₁, esculin (vitamin P active compound), thiamine (vitamin B₁), nicotinic acid (niacin), pyridoxine, pyridoxal, pyridoxamine, (vitamin B₆), pantothenic acid, biotin, folic acid and cobalamine (vitamin B₁₂), particularly preferably vitamin A palmitate, vitamin C and derivatives thereof, DL-α-tocopherol, tocopherol E acetate, nicotinic acid, pantothenic acid and biotin. In the case of cosmetic application, vitamins are usually added with the mixture according to the invention in ranges from 0.01% by weight to 5.0% by weight, based on the total weight.

The retinoids described are at the same time also effective anti-cellulite active compounds. A likewise known anti-cellulite active compound is caffeine.

The preparations to be employed may comprise skin-lightening active compounds. Skin-lightening active compounds can in principle be all active compounds known to the person skilled in the art. Suitable for combination are commercially available melanogenesis inhibitors, such as, for example, ascorbic acid and derivatives thereof, aloesin, niacinamide, emblica, elagic acid, mulberry extract, kojic acid, liquorice extract, rucinol, hydroquinone, azelaic acid, arbutin, magnesium ascorbyl phosphate, lactic acid, butylphenylmethoxyphenylpropandiol (available as RonaCare® PristineBright® or RonaCare®PristineBright® liquid from Merck) or the like.

The preparations may preferably comprise assistants, such as, for example, cosmetic oils (for example Caprylic/Capric Triglycerides, C12-15 alkyl Benzoate, isopropyl myristate, arylalkyl benzoates, such as, for example, phenethyl benzoate (X-Tend 226) or oil components of the Cosmacol brand, such as Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), or polar-protic assistants (for example propylene glycol, glycerol, isopropanol, ethanol) or so-called solubilisers (for example Butylphthalimide, Isopropylphthalimide, Dimethylisosorbide). Very particularly preferred cosmetic oils are C12-C13 Alkyl Lactate, commercially available as Cosmacol ELI and phenethyl benzoate, commercially available as X-Tend 226.

The preparations as described before may be synthesized as known in the art with the aid of techniques which are well known to the person skilled in the art. Suitable vehicles and assistants or fillers are described in detail in the following part.

Preparations suitable for external use, for example can be applied or sprayed onto the skin as cream or milk (O/W, W/O, O/W/O, W/O/W, W/Si), as lotion or emulsion, in the form of oily-alcoholic, oily-aqueous or aqueous-alcoholic gels or solutions. They can be in the form of solid sticks or formulated as aerosol. They can be in the form of shampoo, shower gel, cleansing milk or serum.

The following, for example, may be mentioned as application form of the preparations to be employed: solutions, suspensions, emulsions, PIT emulsions, pastes, ointments, gels, creams, lotions, powders, oils, aerosols plasters, compresses, bandages and sprays.

Preferred assistants originate from the group of preservatives, stabilisers, solubilisers, colorants, odour improvers, thickeners, plasticisers, humectants, interface-active agents, emulsifiers, preservatives, antifoaming agents, perfumes, waxes, lanolin, propellants and other ingredients usually used in cosmetics.

Ointments, pastes, creams and gels may comprise the customary vehicles which are suitable for topical application, for example animal and vegetable fats, waxes, paraffins, starch, tragacanth, cellulose derivatives, polyethylene glycols, silicones, bentonites, silica, talc and zinc oxide, or mixtures of these substances.

Powders and sprays may comprise the customary vehicles, for example lactose, talc, silica, aluminium hydroxide, calcium silicate and polyamide powder, or mixtures of these substances. Sprays may additionally comprise the customary readily volatile, liquefied propellants, for example chlorofluorocarbons, propane/butane or dimethyl ether. Compressed air can also advantageously be used.

Solutions and emulsions may comprise the customary vehicles, such as solvents, solubilisers and emulsifiers, for example water, ethanol, isopropanol, ethyl carbonate, ethyl acetate, benzyl alcohol, benzyl benzoate, propylene glycol, 1,3-butyl glycol, oils, in particular cottonseed oil, peanut oil, wheatgerm oil, olive oil, castor oil and sesame oil, XTend 226, glycerol fatty acid esters, polyethylene glycols and fatty acid esters of sorbitan, or mixtures of these substances.

Suspensions may comprise the customary vehicles, such as liquid diluents, for example water, ethanol or propylene glycol, suspension media, for example ethoxylated isostearyl alcohols, polyoxyethylene sorbitol esters and polyoxyethylene sorbitan esters, microcrystalline cellulose, aluminium metahydroxide, bentonite, agar-agar and tragacanth, or mixtures of these substances.

Face and body oils may comprise the customary vehicles, such as synthetic oils, such as fatty acid esters, fatty alcohols, silicone oils, natural oils, such as vegetable oils and oily plant extracts, paraffin oils, lanolin oils, or mixtures of these substances.

Further typical cosmetic application forms are also lipsticks, lip-care sticks, powder make-up, emulsion make-up and wax make-up, and sunscreen, pre-sun and after-sun preparations.

The preferred preparation forms also include, in particular, emulsions.

Emulsions are advantageous and comprise, for example, the said fats, oils, waxes and other fatty substances, as well as water and an emulsifier, as usually used for a preparation of this type. Emulsifiers that can be used are, for example, known W/O and O/W emulsifiers. It is advantageous to use further conventional co-emulsifiers in preferred O/W emulsions.

Preferred oils and/or lipids for said topical preparations include: paraffin, isoparaffin, dicaprylyl ether, PPG-15, stearyl ether, beeswax, candelilla wax, carnauba wax, ethylhexyl stearate, caprylic/capric triglycerides, cetyl lactate, stearyl stearate, isononyl isononanoate, octyldodecanol, hexyldecanol, squalene, natural triglycerides such as cherry kernel oil (*Prunus Cerasus*), *Persea Gratissima* oil, *Carthamus Tinctorius* seed oil, *Macadamia Ternifolia* seed oil, cocoa butter (*Theobroma Cacao*), *Butyrospermum Parkii* butter and mixtures thereof.

Preferred absorbing and/or texturizing agents for said preparations include modified maize starch, silica, talc, zinc stearate, magnesium sulfate, zinc oxide, calcium and aluminium borosilicate, starches and derivatives, polyurethanes, and mixtures thereof.

As it becomes evident from further investigation of DHA as explained in the experimental section, it was found that such cosmetic preparations can be used as an agent for controlling tropoelastin expression in living cells of the epidermis and in papillary dermis.

The invention therefore relates to the use of a cosmetic composition comprising dihydroxyacetone as described before, as an agent for controlling tropoelastin expression in living cells of the epidermis and in papillary dermis.

Additionally, it becomes evident from further investigation of DHA as explained in the experimental section, such cosmetic preparations can be used as an agent for controlling the degradation of extracellular matrix components in living cells of the epidermis and in papillary dermis.

The invention therefore relates to the use of a cosmetic composition comprising dihydroxyacetone as described before, as an agent for controlling the degradation of extracellular matrix components in living cells of the epidermis and in papillary dermis.

There is no restriction known for applying the topical preparations or the medical device being able to be applied topically as described before comprising DHA.

Useful is a method for the cosmetic treatment of the skin of a mammal, preferably a human, comprising a step of applying a cosmetic preparation comprising dihydroxyacetone as described above to the skin thus enabling the reaction with amino acids and/or proteins of said skin to protect the skin against infrared irradiation as described before.

Useful is a method for the dermatological or pharmaceutical treatment of the skin of a mammal, preferably a human, comprising a step of applying a dermatological or pharmaceutical preparation comprising dihydroxyacetone or a medical device comprising dihydroxyacetone as described above to the skin thus enabling the reaction with amino acids and/or proteins of said skin to protect the skin against damages caused by infrared irradiation as described before.

Useful is a method for the dermatological or pharmaceutical treatment of the skin of a mammal, preferably a human, comprising a step of applying a dermatological or pharmaceutical preparation comprising dihydroxyacetone or a medical device comprising dihydroxyacetone as described above to the skin thus enabling the penetration into said skin to protect the skin against damages caused by infrared irradiation as described before.

The application is carried out using standard techniques, for example by the application of shampoo, shower gel, cream, cleansing milk, paste, gel, lotion, serum to the skin to be treated, or the dissolution of predetermined amount of DHA in water and the subsequent use of said water thus admixed or of the foam formed for cleansing or skin treatment.

It should be pointed out that variations of the embodiments described in the present invention are covered by the scope of this invention. Any feature disclosed in the present invention may, unless this is explicitly ruled out, be exchanged for alternative features which serve the same purpose or an equivalent or similar purpose. Thus, any feature disclosed in the present invention, unless stated otherwise, should be considered as an example of a generic series or as an equivalent or similar feature.

All features of the present invention may be combined with one another in any manner, unless particular features and/or steps are mutually exclusive. This is especially true of preferred features of the present invention. Equally, features of non-essential combinations may be used separately (and not in combination).

The technical teaching disclosed with the present invention may be abstracted and combined with other examples.

### Examples:

### Experiments investigating infrared A light on human skin explants

The product tested is a solution of 2% DHA in water. This was done to avoid formulation effects of auxiliary ingredients.

### Explant preparation

Human skin explants were prepared on an abdominoplasty coming from a 61-year-old Caucasian woman (Fitzpatrick skin phototype II-III). The explants were kept in survival in BEM culture medium (BIO-EC's Explants Medium) at 37°C in a humid, 5 %-CO₂ atmosphere.
On day 0 (D0), day 3 (D3) and day 4 (D4; 3hours before IR irradiation), day 5 (D5) and day 6 (D6), the product was applied topically followed by 10 minutes of drying. The blank batch (T) did not receive any treatment except the renewal of the culture medium. The culture medium was half renewed (1 ml/well) on day 3 and day 5. Irradiation of the explants were done using an infrared lamp (Dr FISCHER 1000W, 235V, 2500K, 760-3000 nm), for 5040s (720 J/cm²). Water filter and IR760 filter were also used to have the following IR range of 700 nm to 1150 nm. The unirradiated batches were kept in HBSS (Hank's Balanced Saline Solution) in the dark. At the end of the irradiation, all the explants were put back in 2 ml of fresh BEM culture medium.
The explants temperature was measured before and after IR-A irradiation. To avoid temperature-driven side-effects, the explants temperature was maintained at 37°C (max. 39°C) by specially adapted refreshing system.

### MMP1 immunostaining

MMP1 immunostaining has been realized on paraffin sections with a polyclonal anti-MMP1 antibody (Sigma, ref. M4696) diluted at 1:100 in PBS-BSA 0,3%- non-ionic surfactant Tween 20 at 0.05% for 1h at room temperature, using Vectastain Kit Vector amplifier system avidin/biotin, and revealed by VIP, a violet substrate of peroxidase (Vector Laboratories, ref. SK-4600).
The immunostaining was performed using an automated slide processing system (Autostainer, Dako) and assessed by microscopical observation.

### Tropoelastin

Tropoelastin immunostaining has been realized on frozen sections with a monoclonal anti- tropoelastin antibody (Chemicon, ref. MAB2503, clone 10B8) diluted at 1:200 in PBS-BSA 0,3% overnight at 4°C, using Vectastain Kit Vector amplifier system avidin/biotin and revealed by AF488 (Lifetechnologies, ref. A11001). The nuclei were counterstained by propidium iodide.
The immunostaining was assessed by microscopical observation.

### Tropoelastin Results

Table 1 summarizes the staining of tropoelastin in papillary dermis and gives the percentage of surface occupied by tropoelastin immunostaining on day 7.

**Table 1:**

| Tropoelastin (% surface) | Blank explant at D7 | Irradiated blank at D7 | Irradiated blank + product at D7 |
|---|---|---|---|
| Average | 11.1 | 6.1 | 9.5 |
| SD | 1.4 | 1.4 | 2.2 |

On day 7, the blank of the papillary dermis is positive to tropoelastin immunostaining as shown with 11.1% of the surface.

The IR-A irradiation versus blank at day 7 induced a significant decrease by 45% (p<0.01) of tropoelastin expression in the papillary dermis.
When applying the product comprising DHA on an irradiated explant, it provided a significant tropoelastin increase of 57% versus untreated irradiated explant (p<0.01). Therefore, DHA provides an excellent protection of the explants after irradiation to IR-A.

Tropoelastin levels after IR-A irradiation and DHA treatment are comparable to those of blank untreated explant. From a statistical perspective, they are not different (p>0.1).

MMP1 Results epidermis: On day 5 on the blank batch 8.2% of the epidermis is positive to MMP1 immunostaining. The surface percentage positive to MMP1 in the living epidermis is shown below in Table 2.

**Table 2:**

| MMP1 in the epidermis (% surface) | Blank explant at D5 | Irradiated blank at D5 | Irradiated blank + product at D5 |
|---|---|---|---|
| Average | 8.2 | 25.3 | 8.4 |
| SD | 3.9 | 4.5 | 0.9 |

After irradiations to IR-A, the percentage of surface occupied by MMP-1 in the epidermis after treatment with DHA is 8.4% (surface irradiated blank) whereas the percentage of surface occupied by MMP-1 on irradiated untreated blank is 25.3%. This corresponds to a very significant decrease of 65% versus irradiated blank at day 5 (p<0.01). The product (2% DHA in water) provides full protection of the explants against MMP-1 damages after irradiation to IR-A as the MMP-1 levels on untreated non-irradiated explants and on irradiated treated explants are not different from a statistical perspective.

MMP1 Results papillary dermis: On day 5 on the blank batch, 7.0% of the papillary dermis is positive to MMP1 immunostaining. The surface percentage positive to MMP1 in the living papillary dermis is shown here in Table 3.

**Table 3:**

| MMP1 in the papillary dermis (% surface) | Blank explant at D5 | Irradiated blank at D5 | Irradiated blank + product at D5 |
|---|---|---|---|
| Average | 7.0 | 10.2 | 5.9 |
| SD | 1.1 | 1.1 | 2.1 |

On the blank batch at day 57.0% of the papillary dermis is positive to MMP1 immunostaining. The IR-A irradiations (irradiated untreated blank versus non-irradiated blank) induce a significant increase of 45% (p<0.01) of MMP-1 expression in the papillary dermis, in comparison with the blank batch (D5).

After IR-A irradiation and after treatment with the product comprising DHA, the percentage of surface occupied by MMP-1 in the papillary dermis is 5.9% whereas the percentage of surface occupied by MMP-1 after irradiation with IR-A and without DHA treatment is 10.2%. This corresponds to a very significant decrease by 42% (p<0.01).

The product (2% DHA in water) provides full protection of the irradiated explants. Levels of MMP-1 in the dermis on untreated non-irradiated explants and on irradiated explants are not different from a statistical perspective (p=0.21).

### Formulation Examples:

### Example 1: Biphasic composition

| **Ingredients** | **Art. No.** | **INCI** | **[%]** |
|---|---|---|---|
| **A** | | | |
| DHA Rapid | 1.00155 (1) | DIHYDROXYACETONE, TROXERUTIN | 3.00 |
| RonaCare® Ectoin | 1.30200 (1) | ECTOIN | 0.30 |
| Ronastar® Golden Sparks | 1.17042 (1) | CALCIUM ALUMINUM BOROSILICATE, SILICA, CI 77891, TIN OXIDE | 0.05 |
| Glycerol 85% | 1.04091 (1) | GLYCERIN, AQUA (WATER) | 2.00 |
| 1,2-Propanediol | 1.07478 (1) | PROPYLENE GLYCOL | 4.00 |
| Arlasolve DMI | (2) | DIMETHYL ISOSORBIDE | 3.00 |
| RonaCare® Sodium Chloride | 1.32260 (1) | SODIUM CHLORIDE | 0.20 |
| Natpure COL Brown LC816 | (3) | CARAMEL | 0.10 |
| Water, demineralized | | AQUA | ad 100 |

| **B** | | | |
|---|---|---|---|
| Cetiol® OE | (4) | DICAPRYLYL ETHER | 25.00 |

| **C** | | | |
|---|---|---|---|
| Preservatives | | | q.s. |
| Fragrance | PARFUM | | q.s. |

Procedure: Weigh all raw materials of phase A and mix until homogeneous. Add phase B to phase A while stirring. Afterwards add phase C while stirring.

Suppliers: (1) Merck KGaA, Darmstadt, Germany/EMD Performance Materials, (2) Croda , (3) S. Goldmann GmbH & Co. KG, (4) BASF AG

**Example 2: O/W emulsion**

| **Ingredients** | **Art. No.** | **INCI (EU)** | **[%]** |
|---|---|---|---|
| **A** | | | |
| RonaCare® Bronzyl™ | 1.32250 (1) | DIHYDROXY METHYLCHROMONYL PALMITATE | 0.20 |
| Eusolex® OCR | 1.05377 (1) | OCTOCRYLENE | 10.00 |
| Eusolex® 9020 | 1.05844 (1) | BUTYL METHOXYDIBENZOYLMETHAN E | 2.00 |
| RonaCare® AP | 1.30163 (1) | BIS-ETHYLHEXYL HYDROXYDIMETHOXY BENZYLMALONATE | 1.00 |
| Tego® Care PBS 6 MB | (2) | POLYGLYCERYL-6 STEARATE, POLYGLYCERYL-6 BEHENATE | 3.00 |
| Lanette® 18 | (3) | STEARYL ALCOHOL | 2.00 |
| Paracera M | (4) | CERA MICROCRISTALLINA | 1.00 |
| Cetiol® 868 | (3) | ETHYLHEXYL STEARATE | 2.00 |
| Schercemol™ OLO Ester | (5) | OLEYL OLEATE | 2.00 |
| Miglyol® 812 N | (6) | CAPRYLIC/CAPRIC TRIGLYCERIDE | 1.00 |
| Xiameter® PMX-200 Silicone | (7) | DIMETHICONE | 0.50 |
| Fluid (100cs) | | | |

| **B** | | | |
|---|---|---|---|
| Ronastar® Golden Lights | 1.17231 (1) | ALUMINA (OR ALTERNATIVELY: SYNTHETIC SAPPHIRE), CI 77891, TIN OXIDE | 3.00 |
| RonaCare® Ectoin | 1.30200 (1) | ECTOIN | 0.30 |
| 1,2-Propanediol | 1.07478 (1) | PROPYLENE GLYCOL | 4.00 |
| Keltrol® CG-RD | (8) | XANTHAN GUM | 0.25 |
| Natrosol 250 HHR | (9) | HYDROXYETHYLCELLULOSE | 0.25 |
| RonaCare® Disodium EDTA | 1.32221 (1) | DISODIUM EDTA | 0.10 |
| Water, demineralized | | AQUA | ad 100 |

| **C** | | | |
|---|---|---|---|
| DHA Plus | 1.10152 (1) | DIHYDROXYACETONE, SODIUM METABISULFITE, MAGNESIUM STEARATE | 3.35 |
| Water, demineralized | | AQUA | 10.00 |

| **D** | | | |
|---|---|---|---|
| Preservatives | | | q.s. |
| Fragrance | | PARFUM | q.s. |

Procedure: Phase B: disperse Keltrol and Natrosol in water. Heat phase A and phase B to 80°C. Add phase A to phase B slowly while stirring. Homogenize. Cool down while stirring. Add phase C and D at approx. 40°C.

Suppliers: (1) Merck KGaA, Darmstadt, Germany/EMD Performance Materials, (2) Evonik Nutrition & Care GmbH, (3) BASF AG, (4) Paramelt, (5) Lubrizol, (6) IOI Oleo GmbH, (7) Biesterfeld, (8) RAHN GmbH, (9) Aqualon GmbH

**Example 3: O/W emulsion**

| **Ingredients** | **Art. No.** | **INCI (EU)** | **[%]** |
|---|---|---|---|
| **A** | | | |
| RonaCare® Bronzyl™ | 1.32250 (1) | DIHYDROXY METHYLCHROMONYL PALMITATE | 0.20 |
| Eusolex® OCR | 1.05377 (1) | OCTOCRYLENE | 10.00 |
| Eusolex® 9020 | 1.05844 (1) | BUTYL METHOXYDIBENZOYLM ETHANE | 2.00 |
| RonaCare® AP | 1.30163 (1) | BIS-ETHYLHEXYL HYDROXYDIMETHOXY BENZYLMALONATE | 1.00 |
| Tego® Care PBS 6 MB | (2) | POL YGL YCERYL-6 STEARATE, POL YGL YCERYL-6 BEHENATE | 3.00 |
| Lanette® 18 | (3) | STEARYL ALCOHOL | 2.00 |
| Paracera M | (4) | CERA MICROCRISTALLINA | 1.00 |
| Cetiol® 868 | (3) | ETHYLHEXYL STEARATE | 2.00 |
| Schercemol™ OLO Ester | (5) | OLEYL OLEATE | 2.00 |
| Miglyol® 812 N | (6) | CAPRYLIC/CAPRIC TRIGLYCERIDE | 1.00 |
| Xiameter® PMX-200 Silicone Fluid (100cs) | (7) | DIMETHICONE | 0.50 |

| **B** | | | |
|---|---|---|---|
| RonaFlair® White Sapphire | 1.17751 (1) | ALUMINA (OR ALTERNATIVELY: SYNTHETIC SAPPHIRE) | 3.00 |
| RonaCare® Ectoin | 1.30200 (1) | ECTOIN | 0.30 |
| 1,2-Propanediol | 1.07478 (1) | PROPYLENE GLYCOL | 4.00 |
| Keltrol® CG-RD | (8) | XANTHAN GUM | 0.25 |
| Natrosol 250 HHR | (9) | HYDROXYETHYLCELLU LOSE | 0.25 |
| RonaCare® Disodium EDTA | 1.32221 (1) | DISODIUM EDTA | 0.10 |
| Water, demineralized | | AQUA | ad 100 |

| **C** | | | |
|---|---|---|---|
| DHA Plus | 1.10152 (1) | DIHYDROXYACETONE, SODIUM METABISULFITE, MAGNESIUM STEARATE | 3.35 |
| Water, demineralized | | AQUA | 10.00 |

| **D** | | | |
|---|---|---|---|
| Preservatives | | | q.s. |
| Fragrance | | PARFUM | q.s. |

Procedure: Phase B: disperse Keltrol and Natrosol in water. Heat phase A and phase B to 80°C. Add phase A to phase B slowly while stirring. Homogenize. Cool down while stirring. Add phase C and D at approx. 40°C.

Suppliers: (1) Merck KGaA, Darmstadt, Germany/EMD Performance Materials, (2) Evonik Nutrition & Care GmbH, (3) BASF AG, (4) Paramelt, (5) Lubrizol, (6) IOI Oleo GmbH, (7) Biesterfeld, (8) RAHN GmbH, (9) Aqualon GmbH

**Example 4: Gel**

| **Ingredients** | **Art. No.** | | **INCI (EU)** | **[%]** |
|---|---|---|---|---|
| **A** | | | | |
| Sepinov P88 | | (1) | SODIUM ACRYLATE/ACRYLOYLDIMETHYLTAURATE /DIMETHYLACRYLAMIDE CROSSPOLYMER | 1.50 |
| Miglyol® 812 N | | (2) | CAPRYLIC/CAPRIC TRIGLYCERIDE | 6.50 |
| Tegosoft® Liquid | | (3) | CETEARYL ETHYLHEXANOATE | 6.50 |
| Xiameter® | | (4) | CYCLOPENTASILOXANE, | 1.20 |
| PMX-0345 | | | CYCLOHEXASILOXANE | |
| Xiameter® | | (4) | | 0.50 |
| PMX-200 Silicone Fluid | | | DIMETHICONE | |
| (100cs) | | | | |
| **B** | | | | |
| 1,2-Propanediol | 1.07478 | (5) | PROPYLENE GLYCOL | 3.00 |
| Water, demineralized | | | AQUA | ad 100 |
| **C** | | | | |
| DHA Rapid | 1.00155 | (5) | DIHYDROXYACETONE, TROXERUTIN | 3.00 |
| Water, demineralized | | | AQUA | 10.0 0 |
| **D** | | | | |
| Preservatives | | | | q.s. |

Procedure: Add phase A slowly with vigorous stirring to phase B. Homogenize. Afterwards add phase C and D.

Suppliers: (1) Seppic GmbH, (2) IOI Oleo GmbH, (3) Evonik Nutrition & Care GmbH (4) Biesterfeld, (5) Merck KGaA, Darmstadt, Germany/EMD Performance Materials

**Example 5: Rinse off Wash & Tan**

| **Ingredients** | **Art. No.** | | **INCI (EU)** | **[%]** |
|---|---|---|---|---|
| **A1** | | | | |
| Dihydroxyacetone | 1.10150 | (1) | DIHYDROXYACETONE | 10.00 |
| Isopentyldiol | | (2) | ISOPENTYLDIOL | 3.00 |
| Water, demineralized | | | AQUA | ad 100 |
| **A2** | | | | |
| Cekol® 2000 | | (2) | CELLULOSE GUM | 0.50 |
| **A3** | | | | |
| Blanova® TENS APG 818 | | (2) | COCO-GLUCOSIDE, AQUA | 2.00 |
| **B** | | | | |
| Sweet Almond Oil | | (3) | PRUNUS DULCIS | 5.00 |
| Dermosoft® Octiol | | (4) | CAPRYLYL GLYCOL | 1.00 |
| **C** | | | | |
| Blanova® PAA-L | | (2) | POLYACRYLAMIDE, C13-14 ISOPARAFFIN, LAURETH-7, AQUA | 3.00 |
| **D** | | | | |
| Fragrance | | | PARFUM | q.s. |

Procedure: Disperse phase A2 in water in phase A1 until homogenous. Add phase 3 mixing until homogenous. Mix phase B gentle heating may be required. Add phase B to phase A with stirring. Add phase C mixing until homogenous. Adjust the pH using citric acid solution (30%) to about pH 4-5. Add phase D and mix until homogenous.

Suppliers: (1) Merck KGaA, Darmstadt, Germany/EMD Performance Materials, (2) Azelis Germany GmbH, (3) Gustav Heess GmbH, (4) Dr. Straetmans

**Example 6: O/W emulsion**

| **Ingredients** | **Art. No.** | | **INCI (EU)** | **[%]** |
|---|---|---|---|---|
| **A** | | | | |
| Water, demineralized | | | AQUA | ad 100 |
| Glycerol 85% | 1.04091 | (1) | GLYCERIN, AQUA | 5.00 |
| Solagum AX | | (2) | XANTHAN GUM, ACACIA GUM | 0.70 |
| **B** | | | | |
| Montanov™ | | (2) | C14-22 ALCOHOLS, C12-20 ALKYL GLUCOSIDE | 4.00 |
| Montanov™ 14 | | (2) | MYRISTYL ALCOHOL, MYRISTYL GLUCOSIDE | 2.00 |
| LIPOCIRE A SG | | (3) | C10-18 TRIGLYCERIDES | 3.00 |
| Cetiol® SB 45 | | (4) | BUTYROSPERMUM PARKII BUTTER | 5.00 |
| Mango Butter | | (5) | MANGIFERA INDICA (MANGO) SEED BUTTER | 5.00 |
| Crodamol ISIS-LQ-(MV) | | (6) | ISOSTEARYL ISOSTEARATE | 5.00 |
| Compritol 888 CG PELLETS | | (3) | GLYCERYL BEHENATE | 3.00 |
| **C** | | | | |
| Preservatives | | | | q.s. |
| **D** | | | | |
| Dihydroxyacetone | 1.10150 | (1) | DIHYDROXYACETONE | 5.00 |
| Water, demineralized | | | AQUA | 10.00 |

Procedure: Disperse Solagum AX in the water and stir until homogeneous. Add glycerin and stir until homogeneous. Heat phases A and B separately to 80°C. Stir phase B into phase A. Homogenize. Cool down while stirring and add the preservatives. Add phase D while stirring at RT.

Suppliers: (1) Merck KGaA, Darmstadt, Germany/EMD Performance Materials, (2) Seppic, (3) Gattefosse (Deutschland) GmbH, (4) BASF AG, (5) Azelis Germany GmbH, (6) Croda

**Example 7: Solution (water based)**

| **Ingredients** | **Art. No.** | | **INCI (EU)** | **[%]** |
|---|---|---|---|---|
| **A** | | | | |
| Dihydroxyacetone | 1.10150 | (1) | DIHYDROXYACETONE | 10.00 |
| RonaCare® Ectoin | 1.30200 | (1) | ECTOIN | 0.50 |
| 1,2-Propanediol | 1.07478 | (1) | PROPYLENE GLYCOL | 4.50 |
| Glycerol 85% | 1.04091 | (1) | GLYCERIN, AQUA (WATER) | 2.00 |
| Transcutol CG | | (2) | ETHOXYDIGLYCOL | 5.00 |
| Arlasolve DMI | | (3) | DIMETHYL ISOSORBIDE | 1.00 |
| Natpure COL Brown LC816 | | (4) | CARAMEL | 0.30 |
| Water, demineralized | | | AQUA | ad 100 |
| **B** | | | | |
| Tween 20 | 8.17072 | (1) | POLYSORBATE 20 | 1.00 |
| Fragrance | | | PARFUM | q.s. |
| **C** | | | | |
| Preservatives | | | | q.s. |

Procedure: Weigh all raw materials of phase A and mix until homogeneous. Add the dissolved phase B to phase A while stirring. Afterwards add phase C while stirring.

Suppliers: (1) Merck KGaA, Darmstadt, Germany/EMD Performance Materials, (2) Gattefosse (Deutschland) GmbH, (3) Croda, (4) S. Goldmann GmbH & Co. KG

**Example 8: Pump Foam**

| **Ingredients** | **Art. No.** | | **INCI (EU)** | **[%]** |
|---|---|---|---|---|
| **A** | | | | |
| Dihydroxyacetone | 1.10150 | (1) | DIHYDROXYACETONE | 15.00 |
| RonaCare® Ectoin | 1.30200 | (1) | ECTOIN | 0.30 |
| 1,2-Propanediol | 1.07478 | (1) | PROPYLENE GLYCOL | 3.00 |
| Glycerol 85% | 1.04091 | (1) | GLYCERIN, AQUA | 1.00 |
| 1,3-Butanediol | 8.01964 | (1) | BUTYLENE GLYCOL | 1.00 |
| Transcutol CG | | (2) | ETHOXYDIGLYCOL | 1.00 |
| Neo-PCL W/S N | | (3) | TRIDECETH-9, PEG-5 ETHYLHEXANOATE, AQUA | 2.00 |
| Tween 20 | 8.17072 | (1) | POLYSORBATE 20 | 2.00 |
| RonaCare® Disodium EDTA | 1.32221 | (1) | DISODIUM EDTA | 0.05 |
| Water, demineralized | | | AQUA | 50.40 |
| **B** | | | | |
| Xiameter® OFX-5329 Fluid | | (4) | PEG-12 DIMETHICONE | 0.50 |
| C | | | | |
| Unicert Red K7057-J (1% solution) | | (5) | AQUA, CI 17200 | 7.85 |
| Unicert Yellow 08005-J (1% solution) | | (5) | AQUA, CI 19140 | 13.00 |
| Unicert Blue 05601-J (1% solution) | | (5) | AQUA, CI 42090 | 2.90 |
| **D** | | | | |
| Preservatives | | | | q.s. |
| Fragrance | PARFUM | | q.s | |

Procedure: Weigh all raw materials of phase A and mix until homogeneous. Add phase B to phase A while stirring. Afterwards add phase C and D while stirring.

Suppliers: (1) Merck KGaA, Darmstadt, Germany/EMD Performance Materials, (2) Gattefosse (Deutschland) GmbH, (3) Symrise, (4) Biesterfeld, (5) S. Goldmann GmbH & Co. KG

**Example 9: W/Si Gel**

| **Ingredients** | **Art. No.** | | **INCI (CTFA)** | **[%]** |
|---|---|---|---|---|
| **A** | | | | |
| RonaCare® AP | 1.30163 (1) | | BIS-ETHYLHEXYL | 0.50 |
| | | | HYDROXYDIMETHOXY | |
| | | | BENZYLMALONATE | |
| Dow Corning 5225 C | (2) | | CYCLOPENTASILOXANE, | 17.00 |
| | | | PEG/PPG-18/18 | |
| | | | DIMETHICONE | |
| Xiameter® PMX-200 Silicone Fluid (10cs) | | (2) | DIMETHICONE | 5.00 |
| Soybean Oil, refined | (3) | | GLYCINE SOJA | 1.00 |
| | | | (GLYCINE SOJA | |
| | | | (SOYBEAN) OIL) | |
| **B** | | | | |
| Dihydroxyacetone | 1.10150 | (1) | DIHYDROXYACETONE | 4.00 |
| RonaCare® Isoquercetin | 1.30149 | (1) | ISOQUERCETIN | 0.50 |
| RonaCare® Ectoin | 1.30200 | (1) | ECTOIN | 0.30 |
| Ronastar® Silver | 1.17713 (1) | | CALCIUM ALUMINUM | 2.00 |
| | | | BOROSILICATE, SILICA, | |
| | | | CI 77891 (TITANIUM | |
| | | | DIOXIDE), TIN OXIDE | |
| 1,2-Propanediol | 1.07478 | (1) | PROPYLENE GLYCOL | 23.00 |
| Dipropylenglycol | | (4) | DIPROPYLENE GLYCOL | 8.00 |
| RonaCare® Sodium Chloride | 1.32260 | (1) | SODIUM CHLORIDE | 0.75 |
| Ethanol 96% | 1.00971 | (1) | ALCOHOL | 5.00 |
| Caramel Liquid | | (5) | CARAMEL | 0.30 |
| RonaCare® Disodium EDTA | 1.32221 | (1) | DISODIUM EDTA | 0.10 |
| Water, demineralized | | | AQUA (WATER) | 32.55 |
| **C** | | | | |
| Fragrance (q.s.) | PARFUM | | 0.00 | |

Procedure: Combine phase A and add phase B very slowly to phase A while gently stirring. Incorporate phase C. Homogenize.

Suppliers: (1) Merck KGaA, Darmstadt, Germany/EMD Performance Materials, (2) Biesterfeld, (3) Gustav Heess GmbH, (4) BASF AG, (5) S. Goldmann GmbH & Co. KG

**Example 10: O/W emulsion**

| **Ingredients** | **Art. No.** | | **INCI (EU)** | **[%]** |
|---|---|---|---|---|
| **A** | | | | |
| Eusolex® T-EASY | 1.05338 (1) | | TITANIUM DIOXIDE | 5.00 |
| | | | (NANO), SILICA, CETYL | |
| | | | PHOSPHATE | |
| RonaCare® AP | 1.30163 (1) | | BIS-ETHYLHEXYL | 1.00 |
| | | | HYDROXYDIMETHOXY | |
| | | | BENZYLMALONATE | |
| Arlacel™ 165 | (2) | | GLYCERYL STEARATE, | 6.00 |
| | | | PEG-100 STEARATE | |
| Tegin M | | (3) | GLYCERYL STEARATE | 2.50 |
| Parteck® LUB STA 50 | 1.00661 | (1) | STEARIC ACID | 1.00 |
| Shea Butter | (4) | | BUTYROSPERMUM | 3.50 |
| | | | PARKII BUTTER | |
| Dermofeel sensolv | | (5) | ISOAMYL LAURATE | 3.00 |
| Tegosoft XC | | (3) | PHENOXYETHYL | 5.00 |
| | | | CAPRYLATE | |
| Crodamol STS | | (2) | PPG-3 BENZYL ETHER | 3.00 |
| | | | MYRISTATE | |
| Xiameter® PMX-0345 | | (6) | CYCLOPENTASILOXANE, | 3.00 |
| | | | CYCLOHEXASILOXANE | |
| Xiameter® PMX-200 Silicone Fluid (100cs) | | (6) | DIMETHICONE | 0.50 |
| **B1** | | | | |
| RonaCare® Ectoin | 1.30200 | (1) | ECTOIN | 0.30 |
| 1,2-Propanediol | 1.07478 | (1) | PROPYLENE GLYCOL | 3.00 |
| Water, demineralized | | | AQUA | ad 100 |
| **B2** | | | | |
| Keltrol® CG-RD | | (7) | XANTHAN GUM | 0.20 |
| **C** | | | | |
| **Dihydroxyacetone** | 1.10150 | (1) | DIHYDROXYACETONE | 3.00 |
| Water, demineralized | AQUA | | 10.00 | |
| **D** | | | | |
| Preservatives | | | q.s. | |

Procedure: Heat phase A (without Eusolex® T-EASY) to 70-75°C. Disperse Eusolex® T-EASY in phase A. Heat phase A and phase B to 70-75°C. Slowly add phase B to phase A while stirring. Homogenize. Cool down to 40°C and add phase C and D while stirring.

Suppliers: (1) Merck KGaA, Darmstadt, Germany/EMD Performance Materials, (2) Croda, (3) Evonik Nutrition & Care GmbH, (4) H. Erhard Wagner GmbH, (5) Dr. Straetmans, (6) Biesterfeld, (7) RAHN GmbH

**Example 11: O/W emulsion**

| **Ingredients** | **Art. No.** | | **INCI (EU)** | **[%]** |
|---|---|---|---|---|
| **A** | | | | |
| RonaFlair® | *** | (1) | BORON NITRIDE | 5.00 |
| Boroneige® *** | | | | |
| Tego Care PSC 3 | | (2) | POLYGLYCERYL-3-STEARATE/CITRATE | 3.00 |
| Tegin M | | (2) | GLYCERYL STEARATE | 1.25 |
| Lanette® O | | (3) | CETEARYL ALCOHOL | 1.25 |
| Tegosoft liquid | | (2) | CETEARYL | 6.50 |
| | | | ETHYLHEXANOATE | |
| Miglyol 812 N | | (4) | CAPRYLIC/CAPRIC | 6.50 |
| | | | TRIGLYCERIDE | |
| Abil Wax 2434 | | (2) | STEAROXY | 1.20 |
| | | | DIMETHICONE | |
| Xiameter® PMX-200 | | (5) | DIMETHICONE | 0.50 |
| Silicone Fluid (100cs) | | | | |
| **B** | | | | |
| 1,2-Propanediol | 1.07478 | (1) | PROPYLENE GLYCOL | 3.00 |
| Water, demineralized | | | AQUA | ad 100 |
| **C** | | | | |
| **Dihydroxyacetone** | 1.10150 | (1) | DIHYDROXYACETONE | 3.00 |
| Water, demineralized | | | AQUA | 10.00 |
| **D** | | | | |
| Preservatives | | | | q.s. |

| | | | | |
|---|---|---|---|---|
| *** RonaFlair® Boroneige® SF-3 (Art. 1.17774) or RonaFlair® Boroneige® SF-15 (Art. 1.17778) | | | | |

Procedure: Heat phase A and phase B to 80°C. Add phase A slowly to phase B while stirring. Homogenize. Cool down while stirring and add phase C and D at 40°C.

Suppliers: (1) Merck KGaA, Darmstadt, Germany/EMD Performance Materials, (2) Evonik Nutrition & Care GmbH, (3) BASF AG, (4) Sasol Germany GmbH, (5) Biesterfeld

**Example 12: O/W emulsion**

| **Ingredients** | **Art. No.** | **INCI (EU)** | **[%]** |
|---|---|---|---|
| **A** | | | |
| Tego Care PSC 3 | (2) | POLYGLYCERYL-3-STEARATE/CITRATE | 3.00 |
| Tegin M | (2) | GLYCERYL STEARATE | 1.25 |
| Lanette® O | (3) | CETEARYL ALCOHOL | 1.25 |
| Tegosoft liquid | (2) | CETEARYL ETHYLHEXANOATE | 6.50 |
| Miglyol 812 N | (4) | CAPRYLIC/CAPRIC TRIGLYCERIDE | 6.50 |
| Abil Wax 2434 | (2) | STEAROXY DIMETHICONE | 1.20 |
| Xiameter® PMX-200 Silicone Fluid (100cs) | (5) | DIMETHICONE | 0.50 |

| **B** | | | |
|---|---|---|---|
| RonaFlair® Fines | 1.17022 (1) | BISMUTH OXYCHLORIDE | 5.00 |
| 1,2-Propanediol | 1.07478 (1) | PROPYLENE GLYCOL | 3.00 |
| Water, demineralized | | AQUA | ad 100 |

| **C** | | | |
|---|---|---|---|
| **Dihydroxyacetone** | 1.10150 (1) | DIHYDROXYACETONE | 3.00 |
| Water, demineralized | | AQUA | 10.00 |

| **D** | | | |
|---|---|---|---|
| Preservatives | | | q.s. |

Procedure: Heat phase A and phase B to 80°C. Add phase A slowly to phase B while stirring. Homogenize. Cool down while stirring and add phase C and D at 40°C.

Suppliers: (1) Merck KGaA, Darmstadt, Germany/EMD Performance Materials, (2) Evonik Nutrition & Care GmbH, (3) BASF AG, (4) Sasol Germany GmbH, (5) Biesterfeld

**Example 13: O/W emulsion**

| **Ingredients** | **Art. No.** | **INCI (EU)** | **[%]** |
|---|---|---|---|
| **A** | | | |
| RonaFlair® ⁺⁺⁺ | ⁺⁺⁺ (1) | BISMUTH OXYCHLORIDE | 3.00 |
| Tego Care PSC 3 | (2) | POL YGL YCERYL-3-STEARATE/CITRATE | 3.00 |
| Tegin M | (2) | GLYCERYL STEARATE | 1.25 |
| Lanette® O | (3) | CETEARYL ALCOHOL | 1.25 |
| Tegosoft liquid | (2) | CETEARYL ETHYLHEXANOATE | 6.50 |
| Miglyol 812 N | (4) | CAPRYLIC/CAPRIC TRIGLYCERIDE | 6.50 |
| Abil Wax 2434 | (2) | STEAROXY DIMETHICONE | 1.20 |
| Xiameter® PMX-200 Silicone Fluid (100cs) | (5) | DIMETHICONE | 0.50 |

| **B** | | | |
|---|---|---|---|
| 1,2-Propanediol | 1.07478 (1) | PROPYLENE GLYCOL | 3.00 |
| Water, demineralized | | AQUA | ad 100 |

| **C** | | | |
|---|---|---|---|
| **Dihydroxyacetone** | 1.10150 (1) | DIHYDROXYACETONE | 3.00 |
| Water, demineralized | | AQUA | 10.00 |

| **D** | | | |
|---|---|---|---|
| Preservatives | | | q.s. |

| | | | |
|---|---|---|---|
| +++ RonaFlair® B-50 (Art. 1.17060) or RonaFlair® LF-2000 (Art. 1.17077) | | | |

Procedure: Heat phase A and phase B to 80°C. Add phase A slowly to phase B while stirring. Homogenize. Cool down while stirring and add phase C and D at 40°C.

Suppliers: (1) Merck KGaA, Darmstadt, Germany/EMD Performance Materials, (2) Evonik Nutrition & Care GmbH, (3) BASF AG, (4) Sasol Germany GmbH, (5) Biesterfeld

**Example 14: O/W emulsion**

| **Ingredients** | **Art. No.** | **INCI (EU)** | **[%]** |
|---|---|---|---|
| **A** | | | |
| Tego Care PSC 3 | (2) | POLYGLYCERYL-3-STEARATE/CITRATE | 3.00 |
| Tegin M | (2) | GLYCERYL STEARATE | 1.25 |
| Lanette® O | (3) | CETEARYL ALCOHOL | 1.25 |
| Tegosoft liquid | (2) | CETEARYL ETHYLHEXANOATE | 6.50 |
| Miglyol 812 N | (4) | CAPRYLIC/CAPRIC TRIGLYCERIDE | 6.50 |
| Abil Wax 2434 | (2) | STEAROXY DIMETHICONE | 1.20 |
| Xiameter® PMX-200 Silicone Fluid (100cs) | (5) | DIMETHICONE | 0.50 |

| **B** | | | |
|---|---|---|---|
| RonaFlair® White Sapphire | 1.17751 (1) | ALUMINA (OR ALTERNATIVELY: SYNTHETIC SAPPHIRE) | 5.00 |
| 1,2-Propanediol | 1.07478 (1) | PROPYLENE GLYCOL | 3.00 |
| Water, demineralized | | AQUA | ad 100 |

| **C** | | | |
|---|---|---|---|
| **Dihydroxyacetone** | 1.10150 (1) | DIHYDROXYACETONE | 3.00 |
| Water, demineralized | | AQUA | 10.00 |

| **D** | | | |
|---|---|---|---|
| Preservatives | | | q.s. |

Procedure: Heat phase A and phase B to 80°C. Add phase A slowly to phase B while stirring. Homogenize. Cool down while stirring and add phase C and D at 40°C.

Suppliers: (1) Merck KGaA, Darmstadt, Germany/EMD Performance Materials, (2) Evonik Nutrition & Care GmbH, (3) BASF AG, (4) Sasol Germany GmbH, (5) Biesterfeld

### Brief description of the figures

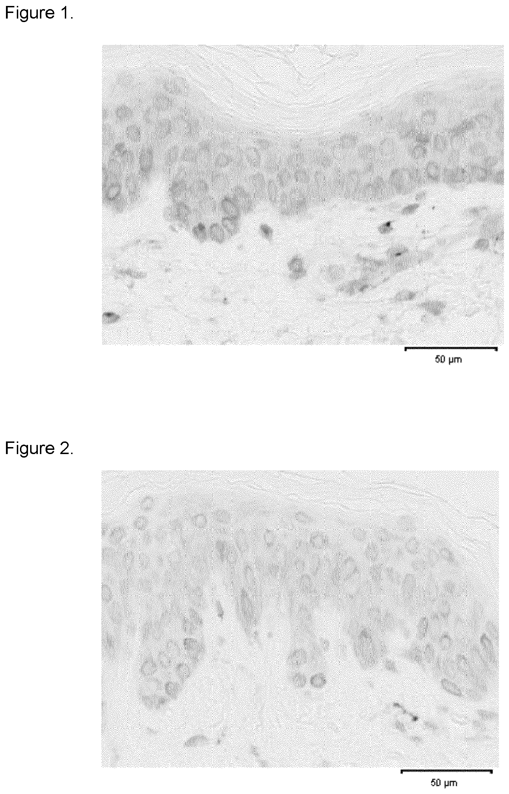

Figure 1 shows the MMP1 immunostaining for the irradiated control explant on day 5.
Figure 2 shows the MMP-1 immunostaining for the treated explant with the product under irradiation on day 5.

## Claims

1. Non-therapeutic, cosmetic use of dihydroxyacetone in a cosmetic composition to be applied on skin, scalp, or hair for the protection of skin, scalp, or hair against infrared irradiation.

2. Use of dihydroxyacetone for the preparation of topical dermatological or pharmaceutical compositions or medical devices to be applied on skin, scalp, or hair for the protection of skin, scalp or hair against damages of infrared irradiation.

3. Use according to claim 1 or 2, wherein dihydroxyacetone protects skin, scalp, or hair against IR-A irradiations between 700 nm and 1400 nm.

4. Use according to claim 1 or 2, wherein dihydroxyacetone protects skin, scalp, or hair against IR-A irradiations between 700 nm and 1150 nm.

5. Dihydroxyacetone as an active ingredient to protect skin, scalp, or hair against damages after infrared irradiations by reacting with the skin, scalp, or hair.

6. Dihydroxyacetone according to claim 5, wherein infrared irradiation is IR-A irradiation between 700 nm and 1400 nm.

7. Dihydroxyacetone according to claim 5, wherein infrared irradiation is IR-A irradiation between 700 nm and 1150 nm.

8. Use of a cosmetic composition comprising dihydroxyacetone as an agent for controlling tropoelastin expression in living cells of the epidermis and in papillary dermis.

9. Use of a cosmetic composition comprising dihydroxyacetone as an agent for controlling the degradation of extracellular matrix components in living cells of the epidermis and in papillary dermis.

10. Dihydroxyacetone as an active ingredient to protect skin, scalp, or hair against damages after infrared irradiations by penetrating the skin, scalp, or hair.

11. Dihydroxyacetone according to claim 10, wherein infrared irradiation is IR-A irradiation between 700 nm and 1400 nm.

12. Dihydroxyacetone according to claim 10, wherein infrared irradiation is IR-A irradiation between 700 nm and 1150 nm.
